(19) Europäisches Patentamt
European Patent Office
Office européen des brevets

(11) Veröffentlichungsnummer: **0 090 297**
**A2**

# (12) EUROPÄISCHE PATENTANMELDUNG

(21) Anmeldenummer: **83102733.9**

(22) Anmeldetag: **19.03.83**

(51) Int. Cl.³: **C 07 D 233/96**

(30) Priorität: **31.03.82 DE 3211911**

(43) Veröffentlichungstag der Anmeldung: **05.10.83**
**Patentblatt 83/40**

(84) Benannte Vertragsstaaten: **DE FR GB IT**

(71) Anmelder: **BAYER AG, Zentralbereich Patente, Marken und Lizenzen, D-5090 Leverkusen 1, Bayerwerk (DE)**

(72) Erfinder: **Findeisen, Kurt, Dr., In der Follmühle 10, D-5068 Odenthal (DE)**
Erfinder: **Buschhaus, Hans-Ulrich, Dr., Morgengraben 2, D-5000 Köln 80 (DE)**
Erfinder: **Bock, Manfred, Dr., Haydnstrasse 18, D-5090 Leverkusen 1 (DE)**
Erfinder: **Mennicken, Gerhard, Dr., Am Wasserturm 16, D-5090 Leverkusen 3 (DE)**

(54) **Verfahren zur Herstellung von N,N'-disubstituierten 5-Acylimino-imidazolidindionen.**

(57) N,N'-disubstituierte 5-Acylimino-imidazolidindione werden durch Umsetzung von N,N'-disubstituierten 5-Trialkylsilylimino-imidazolidindionen mit anorganischen oder organischen Säurehalogeniden oder -anhydriden hergestellt.

EP 0 090 297 A2

0090297

BAYER AKTIENGESELLSCHAFT 5090 Leverkusen, Bayerwerk

Zentralbereich
Patente, Marken und Lizenzen Wr/ABc

# Verfahren zur Herstellung von N,N'-disubstituierten 5-Acylimino-imidazolidindionen

Gegenstand der Erfindung ist ein neues Verfahren zur Herstellung von N,N'-disubstituierten 5-Acylimino-imidazolidindionen der allgemeinen Formel

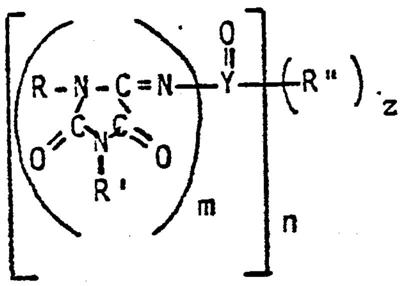

in der

R und R' gleich oder verschieden sind und einen jeweils gegebenenfalls durch Halogen-, $C_1$-$C_4$-Alkyl, Methoxy-, Nitro-, $C_1$-$C_4$-Carbalkoxy-, Nitril- oder Isocyanatgruppen substituierten aliphatischen Kohlenwasserstoffrest mit 1-20 Kohlenstoffatomen, cycloaliphatischen Kohlenwasserstoffrest mit 6-15 Kohlenstoffatomen, aromatischen Kohlenwasserstoffrest mit 6-15 Kohlenstoffatomen oder araliphatischen Kohlenwasserstoffrest mit 7-15 Kohlenstoffatomen darstellen,

Y für Kohlenstoff, Schwefel oder Phosphor steht,

R" für einen jeweils gegebenenfalls inerte Substituenten aufweisenden, n-wertigen aliphatischen Kohlenwasserstoffrest mit 2 bis 20, vorzugsweise 2 bis 6 Kohlenstoffatomen, cycloaliphatischen Kohlenwasserstoffrest mit 3 bis 15, vorzugsweise 6 Kohlenstoffatomen, aromatischen Kohlenwasserstoffrest mit 6 bis 15, vorzugsweise 6 bis 10 Kohlenstoffatomen, araliphatischen Kohlenwasserstoffrest mit 7 bis 15, vorzugsweise 7 bis 8 Kohlenstoffatomen,

. Alkoxyrest mit 1 bis 4 Kohlenstoffatomen, einen Phenoxyrest oder einen Rest steht, wie er durch Entfernung der Chlorcarbonyl-Gruppen von einem n-funktionellen organischen Carbamidsäurechlorid mit insgesamt 2 bis 10 Kohlenstoffatomen erhalten wird,

m und n jeweils für ganze Zahlen von 1 bis 3, vorzugsweise 1 oder 2 stehen und

z 0 oder 1 bedeutet,

dadurch gekennzeichnet, daß N,N'-disubstituierte 5-Trialkylsilylimino-imidazolidindione der allgemeinen Formel

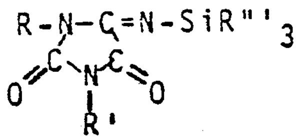

wobei R und R' die oben angeführte Bedeutung haben und R"' für einen aliphatischen Kohlenwasserstoffrest mit 1 bis 6 Kohlenstoffatomen oder aromatischen Kohlenwasserstoffrest mit 6 bis 15 Kohlenstoffatomen steht,

mit anorganischen oder organischen Säurehalogeniden oder Anhydriden der allgemeinen Formel

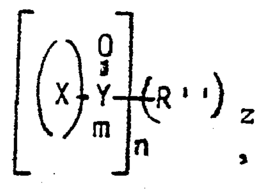

,

wobei Y, R", m, n und z die oben angeführte Bedeutung haben und

X für Halogen oder im Falle von Y = C und m und n jeweils = 1 auch für einen Säurerest $R''-\underset{\overset{\|}{O}}{C}-O-$ steht, wobei in diesem Fall R" für einen einwertigen Kohlenwasserstoffrest der oben genannten Art steht,

umgesetzt werden.

5-Acylimino-imidazolidindione sind an sich bekannt (DE-OS 1 936 157). Wie dieser Lehre zu entnehmen ist, werden zunächst in völlig getrennten Verfahren aus Isocyanaten durch Umsetzung mit Blausäure zunächst 1-Cyanoformamide hergestellt, die in einer zweiten Stufe mit Isocyanaten zu 5-Imino-imidazolidindionen umgesetzt und anschließend mit Ketenen oder Isocyanaten in 5-Acylimino-imidazolidindione umgewandelt werden.

Nachteilig an dem genannten Verfahren ist die Verwendung von Blausäure, die nur unter Vorsichtsmaßnahmen durchgeführt werden kann. Des weiteren ist die Verwendung der nur aufwendig herstellbaren Ketene und die Mehrstufigkeit der Umsetzung nachteilig.

Aufgabe der vorliegenden Erfindung war es daher, die genannten Nachteile, insbesondere die Verwendung von Blausäure bzw. 1-Cyanoformamide, bei der Herstellung von 5-Acylimino-imidazolidindionen zu umgehen. Diese Aufgabe konnte mit dem erfindungsgemäßen Verfahren gelöst werden.

Erfindungsgemäß werden bevorzugt als Säurehalogenide solche der Formel

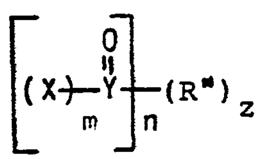

eingesetzt, in denen X für Chlor steht.

Die beim erfindungsgemäßen Verfahren als Ausgangsmaterialien einzusetzenden 5-Trialkylsilylimino-imidazolidindione der oben genannten allgemeinen Formel, für welche R und R' keine Isocyanatsubstituenten aufweisen, können nach bekannten Verfahren (vgl. S. 4 Inada, I. Ojima; J. Organomet. Chem. 169, (1979), 171) durch Umsetzung von Isocyanaten R-NCO oder R'-NCO und/oder Gemischen von Isocyanaten, wobei R und R' die oben angeführte Bedeutung haben, jedoch keine Isocyanatsubstituenten aufweisen, mit Trialkylsilylcyanid $R_3$"'SiCN, wobei R"' die oben angeführte Bedeutung hat, hergestellt werden.

Die als Ausgangsmaterialien einzusetzenden 5-Trialkylsilylimini-imidazolidindione der oben genannten allgemeinen Formel, für welche die Substituenten R und R' jeweils einen Isocyanatsubstituenten aufweisen, werden in Analogie zu den bekannten Verfahren hergestellt, jedoch durch Einsatz eines ca. 5- bis 15-fachen molaren Überschusses an Diisocyanaten R-NCO bzw. R'-NCO (hier weisen die Reste R und R' jeweils einen Isocyanatsubstituenten auf) und destillativer Entfernung des nicht umgesetzten Diisocyanatüberschusses nach Abschluß der Reaktion.

Die so erhaltenen erfindungsgemäß als Ausgangsmaterialien einzusetzenden 5-Trialkylsilylimino-imidazolindione

werden entweder in einem getrennten Reaktionsschritt oder aber bevorzugt in einem Eintopfverfahren unmittelbar nach ihrer Herstellung im Sinne des erfindungsgemäßen Verfahrens nach der Gleichung

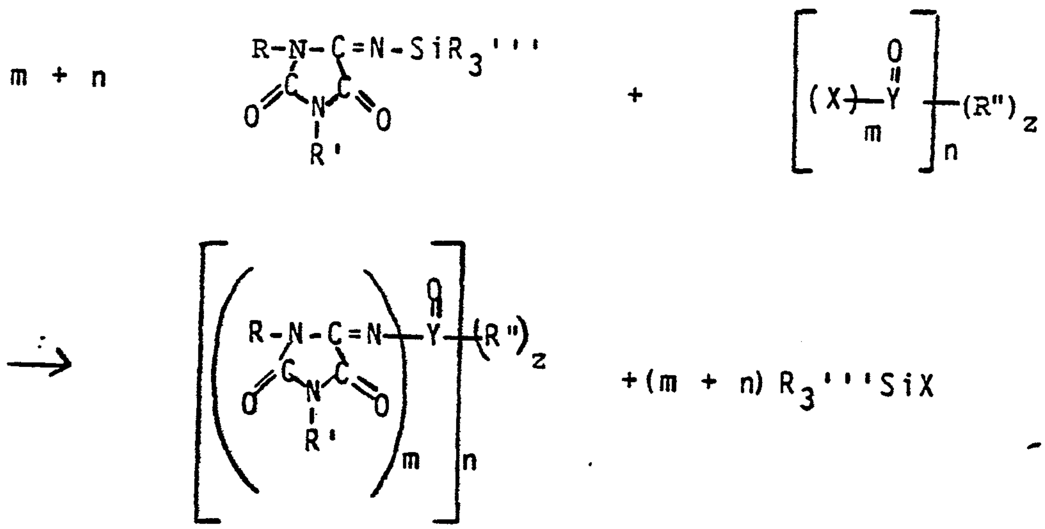

zu 5-Acylimino-imidazolindionen umgesetzt.

Für das Verfahren bevorzugte Isocyanate R-NCO bzw. R'-NCO sind z.B.:

1) Monoisocyanate wie Methylisocyanat, Ethylisocyanat, Propylisocyanat, Isopropylisocyanat, Butylisocyanat, Methoxymethylisocyanat, Stearylisocyanat, 2-Isocyanatoessigsäuremthylester, 6-Isocyanatocapronsäureester, Cyclohexylisocyanat, Phenylisocyanat, 2-Chlorphenylisocyanat, 3-Chlorphenylisocyanat, 4-Chlorphenylisocyanat, 3,4-Dichlorphenylisocyanat, 4-Nitrophenylisocyanat, Benzylisocyanat und andere Isocyanate, wie sie aus der Literatur, z.B. W. Siefken, Annalen der Chemie <u>562</u>, (1949) bekannt sind, und

2) Diisocyanate wie z.B. Tetramethylendiisocyanat, Pentamethylendiisocyanat, Hexamethylendiisocyanat, 1,3-Cyclopentylendiisocyanat, Hexahydroxylylendiisocyanat, 4,4'-Dicyclohexyldiisocyanat, 1,2-Di-(isocyanatomethyl)-cyclobutan, 1,3-bis-(isocyanato-propyl)-2-methyl-2-propylpropan, 1-Methyl-2,4-diisocyanatocyclohexan, 1-Methyl-2,6-diisocyanatocyclohexan, Bis-(4-Isocyanatocyclohexyl)-methan, 1,4-Diisocyanatocyclohexan und 1,3-Diisocyanatocyclohexan, m- und p-Xylyldiisocyanat, 3,3,5-Trimethyl-5-isocyanatomethylcyclohexylisocyanat, 2,6-Diisocyanatocapronsäureester, ß-Isocyanatoethylester- und γ-Isocyanatopropylester der Isocyanatocapronsäure, ganz bevorzugt Hexamethylendiisocyanat, das Isomerengemisch aus 1-Methyl-2,4-diisocyanato-cyclohexan und 1-Methyl-2,6-diisocyanatocyclohexan, Bis-(4-isocyanato-cyclohexyl)-methan, m- und p-Xylylendiisocyanat, 3,3,5-Trimethyl-5-isocyanatomethylcyclohexylisocyanat, methylsubstituiertes Hexamethylen- und Pentamethylendiisocyanat und 2,6-Diisocyanatocapronsäure-$C_1$-$C_4$-alkylester, oder gewünschtenfalls auch aromatische Diisocyanate wie z.B. 1-Methylbenzol-2,4-diisocyanat, 1-Methylbenzol-2,6-diisocyanat, die technischen Toluylen-diisocyanat-Gemische, m- und p-Phenylendiisocyanat, Naphthylendiisocyanat,

Diphenylmethandiisocyanate, Di- und Triisopropylbenzoldiisocyanate, 1-(Isocyanatophenyl)-ethylisocyanat, ferner auch die durch die verschiedensten Substituenten, wie z.B. Alkoxy-, Nitro-, Chlor- oder Brom-substituierten Diisocyanate und andere Diisocyanate, wie sie aus der Literatur, z.B. W. Siefken, Ann. Chem. 562, 6 (1949), bekannt sind.

Bei der Umsetzung der Isocyanate mit Trialkylsilylcyaniden $R_3'''$ SiCN wird besonders bevorzugt Trimethylsilylcyanid verwendet; es können aber auch andere Trialkylsilylcyanide mit R"'-Alkyl $C_2$-$C_{10}$ oder R-Aryl $C_6$-$C_{15}$ oder Mischungen derselben verwendet werden.

Beim erfindungsgemäßen Verfahren können beliebige organische oder anorganische Säurehalogenide oder Anhydride der allgemeinen Formel

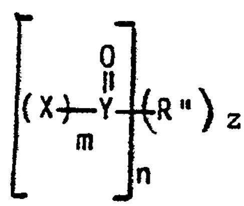

,

wobei X, Y, R", m, n und z die oben angeführte Bedeutung haben, verwendet werden. Als bevorzugt einzusetzende Acyl-Verbindungen seien genannt:

Acetylchlorid, Propionylchlorid, Buttersäurechlorid, Isobuttersäurechlorid, Hexancarbonsäurechlorid, Stearoylchlorid, Cyclohexancarbonsäurechlorid, Monochloracetylchlorid, Dichloracetylchlorid, Trichloracetylchlorid, Benzoylchlorid, Naphthylcarbonsäurechlorid, Chlorameisensäurealkylester, Chlorameisensäurearylester, N-Methylcarbamidsäurechlorid, N-Butylcarbamidsäurechlorid, N,N-Dimethylcarbamidsäurechlorid, N,N-Diethylcarbamidsäurechlorid, Essigsäureanhydrid, Phosgen, Thionylchlorid, Sulfurylchlorid, Phosphortrichlorid, Phosphoroxychlorid, Oxalylchlorid, Bernsteinsäuredichlorid, Adipinsäuredichlorid, Terephthalsäuredichlorid, Phthalsäuredichlorid, Trimesinsäurechlorid.

Bei dem erfindungsgemäßen Verfahren werden besonders bevorzugt Säurechloride eingesetzt, da bei der Umsetzung der Trialkylsilyl-imino-imidazolidindione mit Säurechloriden leichtflüchtige Trialkylsilylchloride entstehen, die sich einerseits leicht aus der Reaktionsmischung entfernen lassen und andererseits nach bekannten Verfahren (vgl. DE-OS 3 018 821) in Trialkylsilylcyanide umgesetzt werden können.

Das erfindungsgemäße Verfahren kann in Substanz oder auch in Anwesenheit eines inerten organischen Lösungsmittels durchgeführt werden. Geeignete inerte Lösungsmittel sind z.B. aliphatische und cycloaliphatische Kohlenwasserstoffe, halogenhaltige Kohlenwasserstoffe, wie Methylenchlorid, Chloroform, Di- und Trichlorethylen, Aromaten, wie Benzol, Toluol, Xylol, die halogenierten Aromaten, wie Chlorbenzol, Dichlorbenzol und Trichlorbenzol, Dioxan, Essigsäureethylester, Ethylglykolacetat, Aceton, Acetonitril, Dimethylformamid und Mischungen dieser Lösungsmittel.

Das erfindungsgemäße Verfahren kann im Temperaturbereich von -40°C bis 300°C, bevorzugt zwischen 0°C und 150°C und besonders bevorzugt zwischen 40°C und 100°C durchgeführt werden.

Bei der Durchführung des erfindungsgemäßen Verfahrens kommen die Ausgangsmaterialien vorzugsweise in etwa stöchiometrischen Mengen, d.h. unter Einhaltung eines Äquivalentverhältnisses von 0,7:1 bis 1,3:1, zum Einsatz. Es ist selbstverständlich auch möglich, insbesondere bei Verwendung von destillativ entfernbaren Säurechloriden, diese in einem beliebig hohen Überschuß einzusetzen, um sie nach Abschluß der erfindungsgemäßen Umsetzung destillativ zu entfernen. Diese Variante ist jedoch weniger bevorzugt.

Die erfindungsgemäßen, Isocyanatgruppen-freien Verfahrensprodukte stellen gemäß der Lehre DE-OS 1 936 157 wertvolle Herbizid-Wirkstoffe dar.

Die erfindungsgemäßen, freie Isocyanatgruppen aufweisenden Verfahrensprodukte stellen eine neue Klasse von organischen Polyisocyanaten dar. Die Tatsache, daß es sich um Verbindungen der eingangs angegebenen allgemeinen Struktur handelt, ergibt sich aus Molekulargewichtsbestimmungen sowie infrarot- (Makromol. Chem. 78 191 (1964)), kernresonanz- und massenspektroskopischen Daten. Die nuen Verbindungen eignen sich als Zwischenprodukte bei der Herstellung von Pflanzenschutzmitteln und insbesondere als wertvolle Ausgangsverbindungen zur Herstellung von Polyurethan-Kunststoffen. Insbesondere stellen die erfindungsgemäßen Polyisocyanate mit aliphatisch gebundenen Isocyanatgruppen wertvolle Ausgangsverbindungen zur Herstellung lichtechter Polyurethan-Lacke und -Folien dar. Die neuen Polyisocyanate weisen eine gute Löslichkeit mit den üblichen Lacklösungsmitteln sowie eine gute Pigmentverträglichkeit auf. Von größter praktischer Bedeutung ist ferner ihr im Vergleich zu den entsprechenden als Ausgangsmaterial verwendeten Diisocyanaten stark verminderter Dampfdruck und ihre hiermit in Zusammenhang stehende physiologische Unbedenklichkeit.

Die aufgeführten Beispiele sollen die Erfindung erläutern, ohne sie jedoch einzuschränken.

0090297

Beispiel 1: 5-Acetylimino-N,N'-dimethylimidazolidindion

106,5 g (0,5 mol) 5-Trimethylsilylimino-N,N'-dimethylimidazolidindion werden mit

39,5 g (0,5 mol) Acetylchlorid und

50 ml Toluol am Rückfluß gekocht. Das entstehende Trimethylsilylchlorid wird über Kopf abdestilliert, restliches Silylchlorid und Toluol bei 15 Torr abgezogen und der feste Rückstand aus 100 ml Toluol umkristallisiert.

Farbloser Feststoff: Ausbeute 54,9 g (60,0 % d.Th.)

| | | | |
|---|---|---|---|
| $C_7H_9N_3O_3$ | ber.: | C 45,9 | gef.: 46,0 |
| | | H 5,0 | 4,9 |
| | | N 22,9 | 22,8 |

Schm.: 136°C.

Beispiel 2: 5-Acetylimino-N,N'-dibutylimidazolidindion aus 5-Trimethylsilylimino-N,N'-dibutylimidazolidindion und Acetylchlorid

356,4 g (1,2 mol) 5-Trimethylsilylimino-N,N'-dibutylimidazolidindion werden bei 130°C vorgelegt und

104,8 g (1,2 mol) Acetylchlorid zugetropft; dabei sinkt die Temperatur auf 100°C.

Es wird 1 Stunde am Rückfluß gekocht; Kopftemperatur zunächst 51°C (Sdp. Acetylchlorid) und nach 1 Std. 58°C (Sdp. Trimethylsilylchlorid).

Das entstehende Silylchlorid wird abdestilliert (128,6 g; 98,3 % d.Th.) und der Rückstand i.V. destilliert.

| | | | |
|---|---|---|---|
| $C_{13}H_{21}N_3O_3$ | ber.: | C 58,4 | gef.: 58,3 |
| | | H 7,9 | 7,9 |
| | | N 15,7 | 15,8 |

Sdp.: 137-139°C/0,1 Torr
Ausbeute: 263,8 g (82,3 % d.Th.)
Farbloser Feststoff, Schmp.: 64-65°C

| | | |
|---|---|---|
| IR: | 1800, 1750 | C=O Ring |
| | 1720 | C=N |
| | 1685 | C=O Acetyl |

| | | | |
|---|---|---|---|
| NMR ($CDCl_3$): | 3,7-3,5 ppm | $N-CH_2$ | 4 P |
| | 2,3 ppm | $CO-CH_3$ | 3 P |
| | 1,6-0,8 ppm | $CH_2-CH_3$ | 14 P |

Beispiel 3: 5-Acetylimino-N,N'-dibutylimidazolidindion aus 5-Trimethylsilylimino-N,N'-dibutylimidazolidindion und Essigsäureanhydrid

89,1 g (0,3 mol) 5-Trimethylsilylimino-N,N'-dibutylimidazolidindion werden mit
30,6 g (0,3 mol) Acetanhydrid gemischt, mit einer Spatelspitze Zinkchlorid versetzt und auf 135°C erwärmt. Entstehendes Trimethylsilylacetat wird zunächst bei Normaldruck (Sdp. 102°C), dann bei 60°C/120 mm Hg abdestilliert.
Menge: 34,5 g (87,4 % d.Th.)
Der tiefbraune Rückstand wird destilliert.
Sdp.: 137-138°C/0,1 Torr

Schmp.: 63°C farbloser Feststoff
Ausbeute: 64,1 g (90,2 % d.Th.)
IR: wie bei Beispiel 2
Der gleiche Versuch ohne Katalyse bzw. unter Verwendung von Pb(II)-Octoat als Katalysator zeigt keine Umsetzung.

Beispiel 4: 5-Phenoxyacylimino-N,N'-dibutylimidazolidindion

89,1 g (0,3 mol) 5-Trimethylsilylimino-N,N'-dibutylimidazolidindion werden mit
47,1 g (0,3 mol) Chlorameisensäurephenylester auf 150°C erwärmt; entstehendes Silylchlorid wird zunächst bei Normaldruck, dann bei 130°C/15 Torr abgetrennt. Es verbleibt ein gelbes Öl.
Ausbeute: 100,3 g (96,9 % d.Th.)
IR: 1810, 1760 C=O Ring
1700 C=O Acyl, C=N

Beispiel 5: 5-Benzoylimino-N,N'-dibutylimidazolidindion

89,1 g (0,3 mol) 5-Trimethylsilylimino-N,N'-dibutylimidazolidindion werden mit
42,3 g (0,3 mol) Benzoylchlorid wie bei Beispiel 2 bei 130°C umgesetzt.
Silylchlorid: 29,8 g (91,1 % d.Th.)
Ausbeute: 98,5 g (99,5 % d.Th.)
hellgelber Feststoff; Schmp. 87°C.

Beispiel 6: Umsetzung von 5-Trimethylsilylimino-N,N'-dibutylimidazolidindion mit Adipinsäuredichlorid

| | |
|---|---|
| 59,4 g | (0,2 mol) 5-Trimethylsilylimino-N,N-dibutylimidazolidindion werden in |
| 100 ml | Toluol mit |
| 18,3 g | (0,2 mol) Adipinsäuredichlorid auf 100°C erwärmt und 4,5 Stunden gerührt. |

Nach Abkühlen auf Raumtemperatur wird das Silylchlorid und Toluol bei 15 Torr abgezogen und der Rückstand aus 100 ml Toluol umkristallisiert.

Schmp.: 134°C farbloser Feststoff

Ausbeute: 40,2 g (71,9 % d.Th.)

| | | | |
|---|---|---|---|
| $C_{28}H_{44}N_6O_6$ | ber.: | C 60,0 | gef.: 59,9 |
| | | H 7,9 | 7,9 |
| | | N 15,0 | 14,9 |

IR: 1800, 1755 C=O Ring
1725 C=N
1680 C=O-Acyl

| NMR ($CDCl_3$): | | | |
|---|---|---|---|
| 3,85-3,5 ppm | N-$CH_2$ | 8 P |
| 2,7 -2,5 ppm | CO-$CH_2$ | 4 P |
| 1,9 -1,2 ppm | -$CH_2$- | 20 P |
| 1,1 -0,85 ppm | -$CH_3$ | 12 P |

Beispiel 7: 5-Trimethylsilylimino-N,N'-dibutylimidazolidindion mit Terephthalsäuredichlorid

89,1 g (0,3 mol) 5-Trimethylsilylimino-N,N'-dibutyl-

imidazolidindion werden in 50 ml o-Dichlorbenzol bei 135°C vorgelegt und eine Lösung aus 30,5 g (0,15 mol) Terephthalsäuredichlorid in 100 ml o-Dichlorbenzol zugetropft. Entstehendes Trimethylchlorsilan wird zunächst bei Normaldruck, dann bei 15 Torr abdestilliert. Man läßt abkühlen, saugt den Feststoff ab und kristallisiert aus 150 ml o-Dichlorbenzol um.

Schmp.: 217°C

Ausbeute: 74,6 g (85,7 % d.Th.)

Beispiel 8: 5-Acetylimino-N,N'-dimethylimidazolidindion

106,5 g (0,5 mol) 5-Trimethylsilylimino-N,N'-dimethylimidazolidindion werden in 50 ml Toluol mit 39,9 g (0,5 mol) Acetylchlorid wie bei Beispiel 2 umgesetzt. Nach dem Einrotieren wird der Feststoff aus 100 ml Toluol umkristallisiert.

Ausbeute: 59,2 g (82,3 % d.Th.)

Schmp.: 136°C

Beispiel 9: 5-Benzoylimino-N,N'-dimethylimidazolidindion
5-Trimethylsilylimino-N,N'-dimethylimidazolidindion

106,5 g (0,5 mol) 5-Benzoylimino-N,N'-dimethylimidazolidindion in 100 ml Toluol werden mit 70,5 g (0,5 mol) Benzoylchlorid wie bei Beispiel 4 umgesetzt. Nach Umkristallisation aus 300 ml Toluol erhält man 79,5 g (65,7 % d.Th.) farblose Kristalle.

Schmp.: 116°C

Beispiel 10: 5-Methacryloylimino-N,N'-di(5-Isocyanato-1,3,3-trimethylcyclohexyl)-imidazolidindion

136 g 5-Trimethylsilylimino-N,N'-di(5-Isocyanato-1,3,3-trimethyl-cyclohexyl-methyl)-imidazolidindion werden bei 130°C unter Stickstoff mit 21,0 g (0,2 mol) Methacrylsäurechlorid $1^1/_2$ h am Rückfluß (Kopftemp.: 58°C) gekocht. Entstehendes Trimethylchlorsilan wird zunächst bei Normaldruck (20,4 g; 93,6 % d.Th.), dann bei 15 Torr abdestilliert
Es verbleibt ein glasartiger Feststoff.
Ausbeute: 124,2 g (89,2 % d.Th.)
Ep.: 85-87°C

Beispiel 11: 5-Acetylimino N,N'-di(5-Isocyanato-1,3,3-trimethyl-cyclohexyl)imidazolidindion

162,9 g 5-Trimethylsilylimino-N,N'-di(5-Isocyanato-1,3,3-trimethyl-cyclohexyl-methyl)-imidazolidindion werden mit 23,7 g (0,3 mol) Acetylchlorid am Rückfluß gekocht. Nach 20 Min. ist eine Kopftemperatur von 55°C erreicht, die nicht weiter ansteigt. Die Mischung aus Acetylchlorid und Trimethylsilylchlorid wird abdestilliert, der Rückstand in 50 ml Toluol aufgenommen und einrotiert.
Ausbeute: 152,0 g (98,8 % d. Th.) zähes Öl, das bei 42°C glasartig erstarrt.

0090297

Beispiel 12:

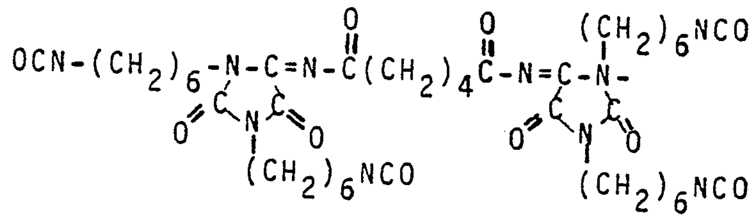

119,3 g 5-Trimethylsilylimino-N,N'-di(6-Isocyanatohexyl)imidazolidin-dion werden bei Raumtemperatur mit

18,3 g (0,1 mol) Adipinsäuredichlorid 2 Tage gerührt; dabei färbt sich der Ansatz rot. Das Trimethylsilylchlorid wird bei 80°C/15 Torr abgezogen. Rückstand: 108,7 g (93,9 % d.Th.)
Der Rückstand wird in 50 g Toluol (68,5 %ige Lösung) aufgenommen und trockene Luft durchgeblasen. Dabei schlägt die Farbe von rot nach orange um.

Beispiel 13:

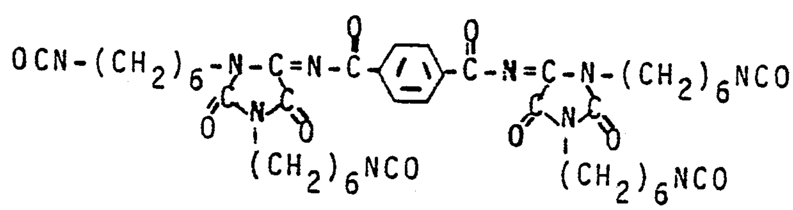

97,0 g 5-Trimethylsilylimino-N,N'-di(6-Isocyanatohexyl)imidazolidindion, verdünnt mit 50 ml Toluol, werden mit einer Lösung aus

20,3 g (0,1 mol) Terephthalsäuredichlorid in 100 ml Toluol umgesetzt. Trimethylsilylchlorid: 16,0 g (73,4 % d. Th.). Nach Abziehen des Toluols bei 80°C/15 Torr verbleiben 90 g (94,2 %) eines hochviskosen Öles.

Beispiel 14: 5-Acetylimino-N,N'-di(6-isocyanatohexyl) imidazolidindion

80,2 g 5-Trimethylsilylimino-N,N'-di(6-isocyanatohexyl)imidazolidindion und
15,8 g (0,2 mol) Acetylchlorid werden 5 h am Rückfluß (Kopftemp. 55°C) gekocht. Nach dem Einrotieren bei 80°C/15 Torr verbleiben 81,0 g (100 % d.Th.) eines orangen Öles.

Patentansprüche

1. Verfahren zur Herstellung von N,N'-disubstituierten 5-Acylimino-imidazolidindionen der allgemeinen Formel

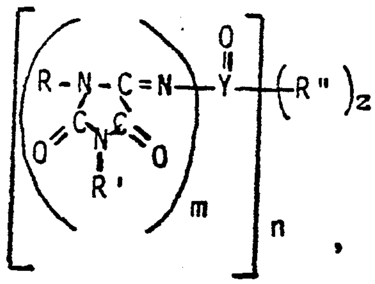

in der

R und R' gleich oder verschieden sind und einen jeweils gegegebenenfalls durch Halogen-, $C_1$-$C_4$-Alkyl, Methoxy-, Nitro-, $C_1$-$C_4$-Carbalkoxy-, Nitril- oder Isocyanatgruppen substituierten aliphatischen Kohlenwasserstoffrest mit 1-20 Kohlenstoffatomen, cycloaliphatischen Kohlenwasserstoffrest mit 6-15 Kohlenstoffatomen, einen aromatischen Kohlenwasserstoffrest mit 6-15 Kohlenstoffatomen oder araliphatischen Kohlenwasserstoffrest mit 7-15 Kohlenstoffatomen darstellen,

Y für Kohlenstoff, Schwefel oder Phosphor steht,

R" für einen jeweils gegebenenfalls inerte Substituenten aufweisenden, n-wertigen aliphatischen Kohlenwasserstoffrest mit 2 bis 20, vorzugsweise 2 bis 6 Kohlenstoffatomen, cycloaliphatischen Kohlenwasserstoffrest mit 3 bis 15, vorzugsweise 6 Kohlenstoffatomen, aromatischen Kohlenwasserstoffrest mit 6 bis 15, vorzugsweise 6 bis 10 Kohlenstoffatomen, araliphatischen Kohlenwasserstoffrest mit 7 bis 15, vorzugsweise 7 bis 8 Kohlenstoffatomen,

Alkoxyrest mit 1 bis 4 Kohlenstoffatomen, einen Phenoxyrest oder einen Rest steht, wie er durch Entfernung der Chlorcarbonyl-Gruppen von einem n-funktionellen organischen Carbamidsäurechlorid mit insgesamt 2 bis 10 Kohlenstoffatomen erhalten wird,

m und n jeweils für ganze Zahlen von 1 bis 3, vorzugsweise 1 bis 2 stehen und

z 0 oder 1 bedeutet,

dadurch gekennzeichnet, daß N,N'-disubstituierte 5-Trialkylsilylimino-imidazolindione der allgemeinen Formel

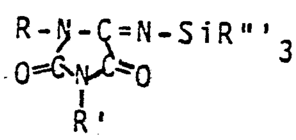

wobei R und R' die oben angeführte Bedeutung haben und R"' für einen aliphatischen Kohlenwasserstoffrest mit 1 bis 6 Kohlenstoffatomen oder aromatischen Kohlenwasserstoffrest mit 6 bis 15 Kohlenstoffatomen steht, mit anorganischen oder organischen Säurehalogeniden oder Anhydriden der allgemeinen Formel

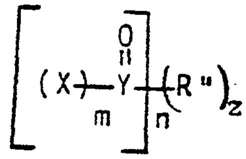

wobei Y, R", m, n und z die oben angeführte Bedeutung haben und

X für Halogen oder im Falle von Y = C und m und n jeweils = 1 auch für einen Säurerest

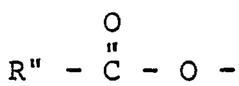

steht, wobei in diesem Falle R" für einen einwertigen Kohlenwasserstoffrest der oben genannten Art steht,

umgesetzt werden.

2. Verfahren gemäß Anspruch 1, dadurch gekennzeichnet, daß als Säurehalogenide solche der Formel

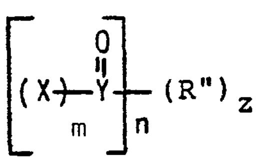,

eingesetzt werden, in denen X für Chlor steht.